# EUROPEAN PATENT APPLICATION

(11) **EP 4 005 634 A1**
(43) Date of publication of application: **01.06.2022**
(21) Application number: 20844175.8
(22) Date of filing: 22.07.2020
(51) Int. Cl.: A61P 9/10, A61P 35/00, C12N 15/113, A61K 31/7088, A61K 31/711

(54) **CHIMERIC MOLECULE, PHARMACEUTICAL COMPOSITION, METHOD FOR CLEAVING TARGET NUCLEIC ACID, AND KIT FOR TARGET NUCLEIC ACID CLEAVAGE OR DIAGNOSIS**

(30) Priority: 24.07.2019 JP 2019136414
(71) Applicant: Tohoku University, Sendai-shi, Miyagi 980-8577 (JP)
(72) Inventor: WADA Takehiko, Sendai-shi, Miyagi 980-8577 (JP)
(74) Representative: Arnold & Siedsma
(86) International application number: PCT/JP2020/028421
(87) International publication number: WO 2021/015234

(57) **Abstract**

A chimeric molecule resulting from fusion of a first nucleic acid or a derivative thereof, which has an ability to bind to a target nucleic acid, with a second nucleic acid or a derivative thereof, which has an ability to bind to the target nucleic acid, and in which a main chain skeleton is anionic, a pharmaceutical composition containing the chimeric molecule, a method for cleaving a target nucleic acid using the chimeric molecule, and a kit for target nucleic acid cleavage or diagnosis including the chimeric molecule.

## Description

### TECHNICAL FIELD

The present invention relates to a chimeric molecule, a pharmaceutical composition, a method for cleaving a target nucleic acid, and a kit for target nucleic acid cleavage or diagnosis.

The present application claims priority based on Japanese Patent Application No. 2019-136414 filed in Japan on July 24, 2019, the contents of which are incorporated herein by reference.

### BACKGROUND ART

In recent years, a nucleic acid drug has been attracting attention as a next-generation molecular target drug as well as an antibody drug. In particular, a nucleic acid drug also targets a disease that cannot be treated with an antibody drug. In addition, the nucleic acid drug has many advantages such that it can be relatively inexpensively supplied by chemical synthesis, and its position as a post low molecular weight drug is being established in the same manner as the antibody drug.

Numerous drug strategies have been reported for nucleic acid drugs. For example, an antisense nucleic acid (ASO) targets a messenger RNA (mRNA), a microRNA (miRNA), an siRNA, or the like involved in disease progression, and recognizes the target in a base sequence selective manner to form a complex, thereby suppressing the function of the target RNA and exhibiting a therapeutic effect. In order for such a nucleic acid drug to effectively exhibit its drug efficacy, 1) high in vivo stability and 2) high specificity for the target nucleic acid and complex stability are required, and the development of a modified oligonucleic acid resulting from chemical modification of a natural DNA/RNA or an artificial oligonucleic acid has been energetically studied.

Although excellent modified or artificial nucleic acids have been reported so far, the expression of a side effect (an off-target effect in a narrow sense) caused by binding to a non-target nucleic acid having a sequence similar to that of a target nucleic acid, and toxicity (an off-target effect in a broad sense) characteristic of a nucleic acid drug independent of recognition of a target nucleic acid has been pointed out as a problem to be solved for practical use, and its reduction and improvement have been studied worldwide.

As a methodology for overcoming the off-target effect in a broad sense, reduction of the dose of a nucleic acid drug has been proposed. However, when the dose is reduced, the amount of a complex formed with a target RNA naturally decreases, and effective drug efficacy expression cannot be expected. Specifically, when using an ASO whose intracellular introduction amount has been reported to be limited to the sub-nM level, a feedback mechanism has been reported not only in a system targeting a mRNA whose expression level is at the sub-µM level as a matter of course, but also in a system targeting a miRNA exhibiting its function when the intracellular expression level is at the nM to pM level, and it has been pointed out that a sufficient therapeutic effect cannot be expected in a drug efficacy expression strategy based on the formation of a 1:1 complex between a target RNA and an ASO.

As a method for solving this problem, a nucleic acid drug having a catalyst-like function utilizing RNase H, which cleaves a target RNA like a catalyst with a small amount of an ASO, has attracted attention (NPL 1).

### Citation List

### Non Patent Literature

NPL 1: Liang, X. et al., Mol. Ther. 2017, 25, 2075

### SUMMARY OF INVENTION

### Technical Problem

An object of the present invention is to provide a chimeric molecule capable of restraining the function of a target nucleic acid at a low concentration and suppressing an off-target effect, a pharmaceutical composition containing the chimeric molecule, a method for cleaving a target nucleic acid using the chimeric molecule, and a kit for target nucleic acid cleavage or diagnosis containing the chimeric molecule.

### Solution to Problem

The present inventors thought that an increase in turnover number is effective in improving the cleavage efficiency of a target RNA by an RNase. From this point of view, the present inventors proposed a design method that contributes to the construction of an oligonucleic acid system capable of rapid dissociation from a complex of a target RNA after cleavage by focusing on the dissociation process after RNA cleavage, and succeeded in a demonstration experiment. The present inventors found out that by utilizing this method, the function of a target nucleic acid can be restrained at a low concentration and an off-target effect can be suppressed, and thus completed the present invention.

The present invention includes the following aspects.
[1] A chimeric molecule resulting from fusion of a first nucleic acid or a derivative thereof, which has an ability to bind to a target nucleic acid, with a second nucleic acid or a derivative thereof, which has an ability to bind to the target nucleic acid, and in which a main chain skeleton is anionic.
[2] The chimeric molecule according to [1], wherein a main chain skeleton of the first nucleic acid or a derivative thereof is neutral or cationic.
[3] The chimeric molecule according to [2], wherein the main chain skeleton of the first nucleic acid or a derivative thereof is an amide skeleton.
[4] The chimeric molecule according to any one of [1] to [3], wherein the main chain skeleton of the second nucleic acid or a derivative thereof is a sugar-phosphate skeleton.
[5] The chimeric molecule according to any one of [1] to [4], wherein the first nucleic acid or a derivative thereof is fused to the 5' end of the second nucleic acid or a derivative thereof.
[6] The chimeric molecule according to any one of [1] to [4], wherein the first nucleic acid or a derivative thereof is fused to the 3' end of the second nucleic acid or a derivative thereof.
[7] The chimeric molecule according to any one of [1] to [6], wherein a complex composed of the chimeric molecule and the target nucleic acid bound to the chimeric molecule specifically binds to a nuclease.
[8] The chimeric molecule according to [7], wherein the nuclease cleaves the target nucleic acid at a fusion part of the first nucleic acid or a derivative thereof and the second nucleic acid or a derivative thereof.
[9] The chimeric molecule according to [8], wherein the melting temperature Tm of both fragments of the target nucleic acid after cleavage with the nuclease is 38°C or lower.
[10] The chimeric molecule according to any one of [7] to [9], wherein the nuclease is ribonuclease H.
[11] The chimeric molecule according to any one of [1] to [10], wherein the first nucleic acid or a derivative thereof is a peptide nucleic acid or a peptide ribonucleic acid or a derivative thereof.
[12] The chimeric molecule according to any one of [1] to [11], wherein the second nucleic acid or a derivative thereof is a DNA.
[13] The chimeric molecule according to any one of [1] to [12], wherein the target nucleic acid is an RNA or a DNA.
[14] A pharmaceutical composition, containing the chimeric molecule according to any one of [1] to [13] as an active ingredient.
[15] The pharmaceutical composition according to [14], wherein the pharmaceutical composition is a cancer or an ischemic brain disease.
[16] A method for cleaving a target nucleic acid, including cleaving a target nucleic acid using the chimeric molecule according to any one of [1] to [13] and a nuclease.
[17] The method for cleaving a target nucleic acid according to [16], wherein the nuclease is ribonuclease H.
[18] A kit for target nucleic acid cleavage or diagnosis, including the chimeric molecule according to any one of [1] to [13] and a nuclease.
[19] The kit according to [18], wherein the nuclease is ribonuclease H.

### Advantageous Effects of Invention

According to the present invention, a chimeric molecule capable of restraining the function of a target nucleic acid at a low concentration and suppressing an off-target effect, a pharmaceutical composition containing the chimeric molecule, a method for cleaving a target nucleic acid using the chimeric molecule, and a kit for target nucleic acid cleavage or diagnosis containing the chimeric molecule can be provided.

### BRIEF DESCRIPTION OF DRAWINGS

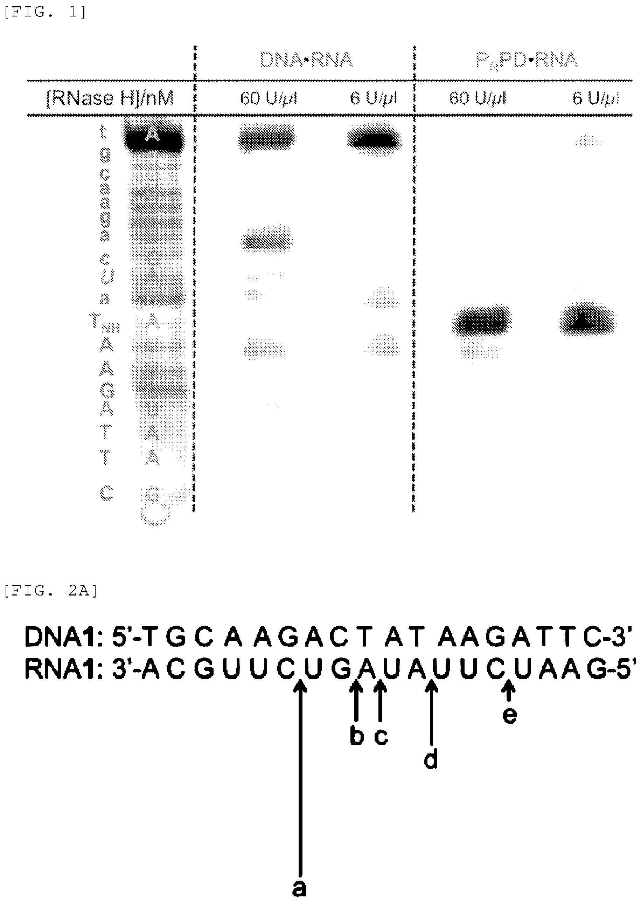

FIG. 1 is a diagram in which cleavage products by RNase H of a P_{R}PD-RNA complex were analyzed with polyacrylamide gel electrophoresis using a system in which an RNA labeled with a fluorescent dye at the 5' end was mixed in an amount 10 times the amount of each. Lane 1 shows the ladder and the base sequence of RNA1, Lane 2 shows a polyacrylamide gel electrophoresis pattern of the cleavage products of RNA1 by RNase H when RNase H was added to a complex of DNA 1 and RNA1 at 60 U/µL, Lane 3 shows the same when RNase H was added to a complex of DNA 1 and RNA1 at 6 U/µL, Lane 4 shows the same when RNase H was added to a complex of P_{R}PD1 and RNA1 at 60 U/µL, and Lane 5 shows the same when RNase H was added to a complex of P_{R}PD1 and RNA1 at 6 U/µL.
FIG. 2A is a view showing the cleavage sites in RNA1 by RNase H in the case of a DNA1-RNA1 complex.

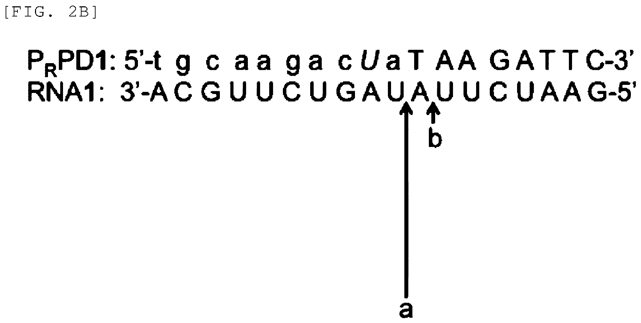

FIG. 2B is a view showing the cleavage sites in RNA1 by RNase H in the case of a P_{R}PD1-RNA1 complex.

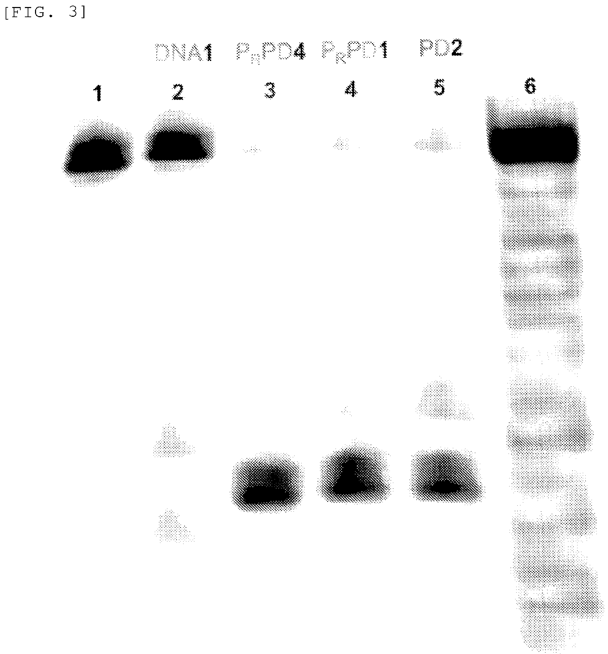

FIG. 3 is a diagram in which cleavage products by RNase H of a complex of DNA1, P_{R}PD4, P_{R}PD1, or PD2 and RNA1 were analyzed with polyacrylamide gel electrophoresis using a system in which an RNA labeled with a fluorescent dye at the 5' end was mixed in an amount 10 times the amount of each. Lane 1 shows a polyacrylamide gel electrophoresis pattern of the cleavage products of RNA1 by RNase H when RNA1 (control), DNA1, P_{R}PD4, P_{R}PD1, and PD2 were added, respectively. Lane 6 shows the ladder and the base sequence of RNA1.

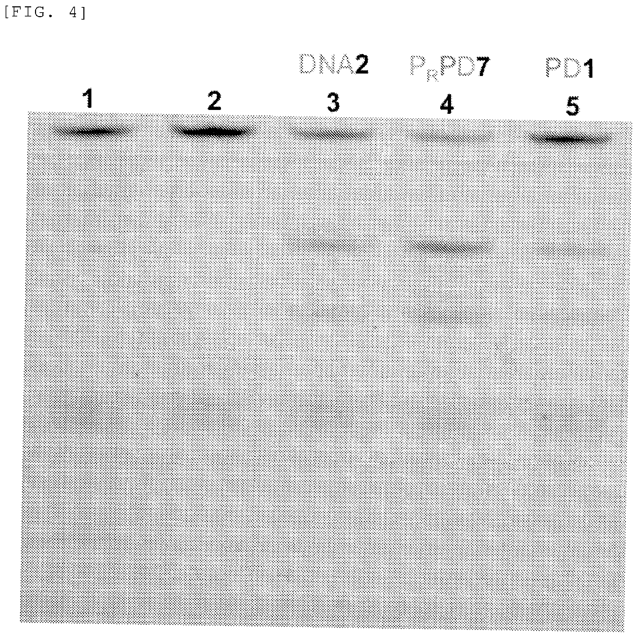

FIG. 4 is a diagram in which cleavage products by RNase H of a complex of DNA2, P_{R}PD7, or PD1 and RNA2 were analyzed with polyacrylamide gel electrophoresis using a system in which an RNA labeled with a fluorescent dye at the 5' end was mixed in an amount 10 times the amount of each. Lane 1 shows the ladder and the base sequence of RNA2, and Lanes 2 to 5 show polyacrylamide gel electrophoresis patterns of the cleavage products of RNA2 by RNase H when RNA2 (control), DNA2, P_{R}PD7, and PD1 were added, respectively.

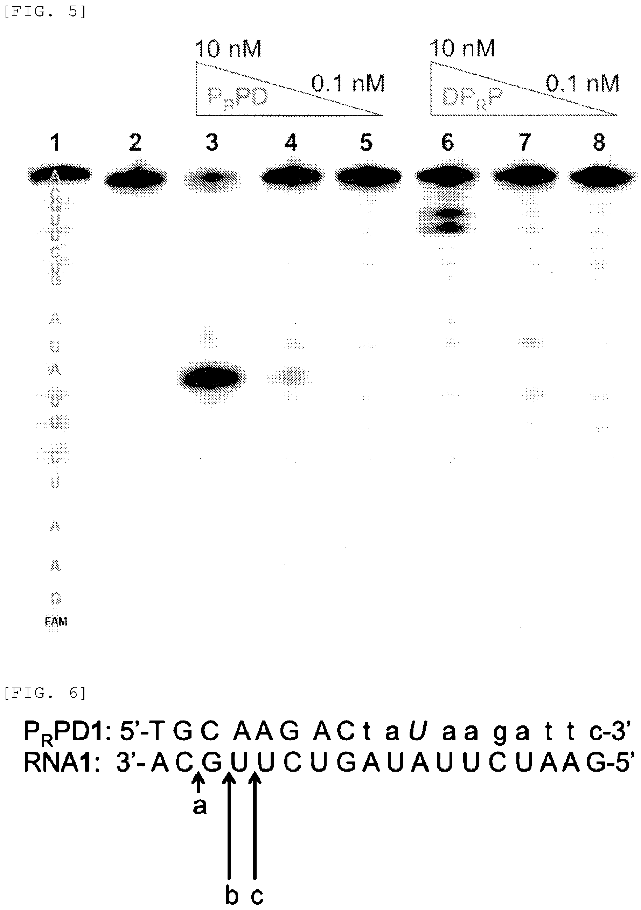

FIG. 5 is a diagram in which cleavage products by RNase H of a complex of DP_{R}P1 or P_{R}PD5 and RNA1 were analyzed with polyacrylamide gel electrophoresis using a system in which an RNA labeled with a fluorescent dye at the 5' end was mixed in an amount 10 times the amount of each. Lane 1 shows the ladder and the base sequence of RNA1, and Lanes 2 to 8 show polyacrylamide gel electrophoresis patterns of the cleavage products of RNA1 by RNase H when RNA1 (control), P_{R}PD5 (10 nM), P_{R}PD5 (1 nM), P_{R}PD5 (0.1 nM), DP_{R}P1 (10 nM), DP_{R}P1 (1 nM), and DP_{R}P1 (0.1 nM) were added, respectively.
FIG. 6 is a view showing the cleavage sites in the RNA of a DP_{R}P1-RNA1 complex by RNase H.

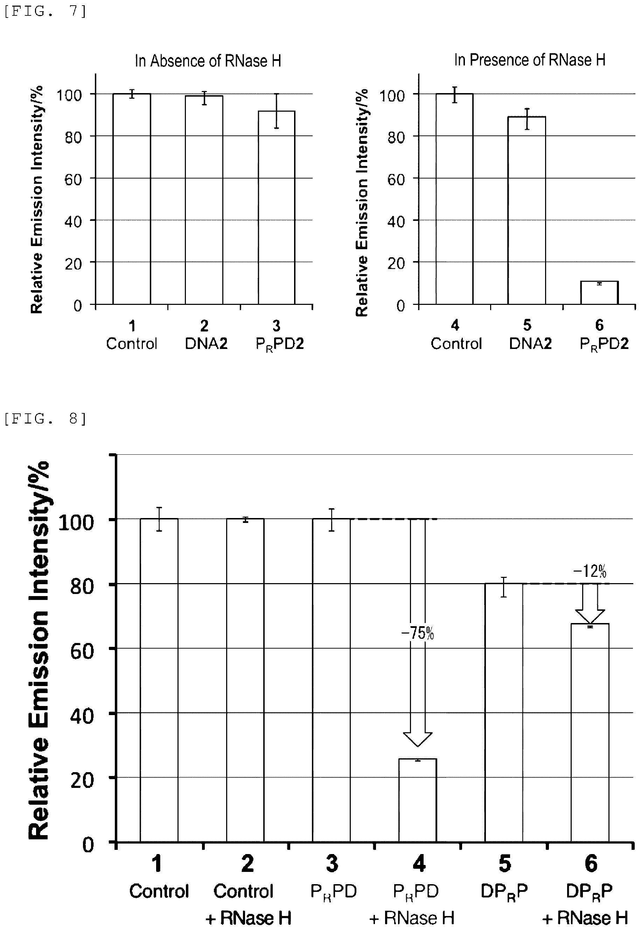

FIG. 7 shows the antisense activity of each of P_{R}PD2 and DNA2 in a cell-free translation system. Lanes 1 to 3 show the results in the absence of RNase H, and Lanes 4 to 6 show the results in the presence of RNase H. Lanes 1 and 4 show a case where a control was added, Lanes 2 and 5 show a case where DNA2 was added, and Lanes 3 and 6 show a case where P_{R}PD2 was added.
FIG. 8 shows the antisense activity of each of P_{R}PD5 and DP_{R}P2 in a cell-free translation system. Lanes 1, 3, and 5 show the results in the absence of RNase H, and Lanes 2, 4, and 6 show the results in the presence of RNase H. Lanes 1 and 2 show a case where a control was added, Lanes 3 and 4 show a case where P_{R}PD5 was added, and Lanes 5 and 6 show a case where DP_{R}P2 was added.

### DESCRIPTION OF EMBODIMENTS

### [Chimeric Molecule]

In a chimeric molecule of the present invention, a first nucleic acid or a derivative thereof, which has an ability to bind to a target nucleic acid, and a second nucleic acid or a derivative thereof, which has an ability to bind to the target nucleic acid, and in which a main chain skeleton is anionic, are fused with each other.

In the present invention, the derivative of the nucleic acid is not particularly limited, but a halogenated derivative, in which a base moiety bound to the nucleic acid is uracil, cytosine, thymine, adenine, guanine, or a purine ring or a pyrimidine ring, a deaminated derivative, or a derivative having a sulfur atom in place of an oxygen atom of each nucleobase, and the like can be exemplified.

In the first nucleic acid or a derivative thereof, which has an ability to bind to the target nucleic acid, a main chain skeleton thereof is preferably neutral or cationic, and more preferably neutral. The neutral main chain skeleton is not particularly limited, but for example, an amide skeleton is exemplified. As the nucleic acid or a derivative thereof having the amide skeleton, for example, a peptide nucleic acid or a derivative thereof (hereinafter peptide nucleic acid is sometimes referred to as PNA), a peptide ribonucleic acid or a derivative thereof (peptide ribonucleic acid: hereinafter sometimes referred to as PRNA), a combination of a PRNA and a PNA (hereinafter also referred to as PNA/PRNA), or the like is exemplified. In a PNA/PRNA, the binding site of a PRNA in a PNA may be any site in the PNA, and it may be bound in the middle of the PNA. For example, it may be a combination such as PNA-PRNA-PNA.

The cationic main chain skeleton is not particularly limited, and for example, an imino skeleton or a phosphate amide or phosphoramidite skeleton, or the like is exemplified. As the nucleic acid or a derivative thereof having each of the above-mentioned skeletons, for example, a morpholino-type nucleic acid, or the like is exemplified.

The main chain skeleton of the second nucleic acid or a derivative thereof, which has an ability to bind to the target nucleic acid, and in which the main chain skeleton is anionic is not particularly limited, but is preferably a sugar-phosphate skeleton. Examples of the nucleic acid or a derivative thereof having the sugar-phosphate skeleton include a ribonucleic acid (ribonucleic acid: hereinafter also referred to as RNA) and a deoxyribonucleic acid (deoxyribonucleic acid: hereinafter also referred to as DNA).

The target nucleic acid is not particularly limited as long as it is a nucleic acid or a derivative thereof having a target sequence to which the chimeric molecule of the present invention can bind, but is preferably an RNA or a DNA, and when the chimeric molecule of the present invention is used as a pharmaceutical composition, the target nucleic acid is preferably an RNA or a DNA encoding a protein that causes a disease to be treated with the pharmaceutical composition.

The first nucleic acid or a derivative thereof may be fused to either of the 3' end and the 5' end of the second nucleic acid or a derivative thereof.

Examples of the chimeric molecule of the present invention resulting from fusion of the first nucleic acid or a derivative thereof with the second nucleic acid or a derivative thereof include a fusion product of a PNA which is the first nucleic acid or a derivative thereof with a DNA which is the second nucleic acid or a derivative thereof (hereinafter also referred to as PNA-DNA chimeric molecule), and a fusion product of a PNA/PRNA which is the first nucleic acid or a derivative thereof with a DNA which is the second nucleic acid or a derivative thereof.
Examples of the fusion product of a PNA/PRNA which is the first nucleic acid or a derivative thereof with a DNA which is the second nucleic acid or a derivative thereof include a chimeric molecule in which a PNA/PRNA is fused to the 5' side of a DNA (hereinafter also referred to as PNA/PRNA-DNA chimeric molecule or P_{R}PD), and a chimeric molecule in which a PNA/PRNA is fused to the 3' side of a DNA (hereinafter also referred to as DNA-PNA/PRNA chimeric molecule or DP_{R}P).

The chimeric molecule of the present invention can be produced as follows.

A PNA-DNA chimeric molecule in which the first nucleic acid is a PNA and the second nucleic acid is a DNA can be produced, for example, as follows.

### (Synthesis of PNA Oligomer)

A PNA oligomer can be synthesized by a known solid phase peptide synthesis method. For example, it can be synthesized according to the steps illustrated below by an Fmoc-solid phase peptide synthesis method (solid phase peptide synthesis: hereinafter also referred to as Fomc-SPPS method) using a benzoyl group-protected Fmoc PNA monomer.

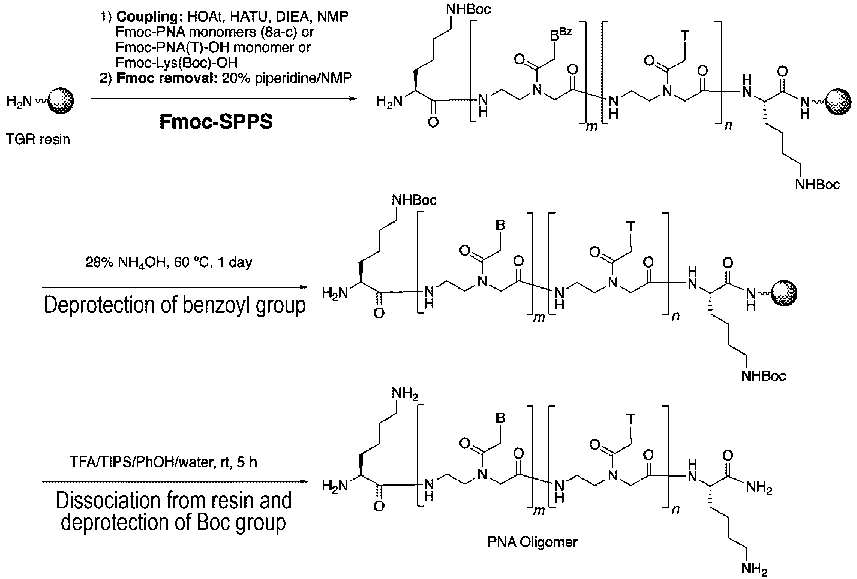

In the above, an N-terminal or C-terminal lysine residue is introduced to improve water solubility and suppress an acyl transfer reaction. The synthesis can be carried out by a known method using a NovaSyn (registered trademark) TGR resin or the like. A condensation reaction of a benzoyl group-protected Fmoc-PNA monomer can be carried out, for example, by performing a treatment for 30 minutes with an NMP (N-methylpyrrolidone) solution containing Fmoc-PNA(Bz)-OH (10 equivalents), HATU (1-[bis(dimethylamino)methyliumyl]-1H-1,2,3-triazolo[4,5-b]pyridine-3-oxide hexafluorophosphate, 10 equivalents), HOAt (1-hydroxy-7-azabenzotriazole, 10 equivalents), and DIEA (N,N-diisopropylethylamine, 20 equivalents). The Fmoc group can be removed by performing a treatment with an NMP solution containing 20% piperidine for 20 minutes. These coupling and Fmoc group removal steps are repeated until the desired sequence is reached. The deprotection of a benzoyl group can be carried out by performing a treatment at 60°C with a 28% aqueous ammonia solution. Finally, the dissociation and deprotection of a Boc group of the lysine residue is carried out by performing a treatment with a TFA (trifluoroacetic acid)/TIPS (triisopropylsilane)/phenol/water (94:3:1:2) mixed solution. The obtained crude product can be purified by reverse-phase HPLC, and the purified product can be identified by ESI-TOF-MS.

### (Synthesis of PNA-DNA Chimeric Molecule)

A PNA-DNA chimeric molecule can be synthesized, for example, as follows by applying a benzoyl-protected Fmoc monomer synthesized by a known method to a target sequence.

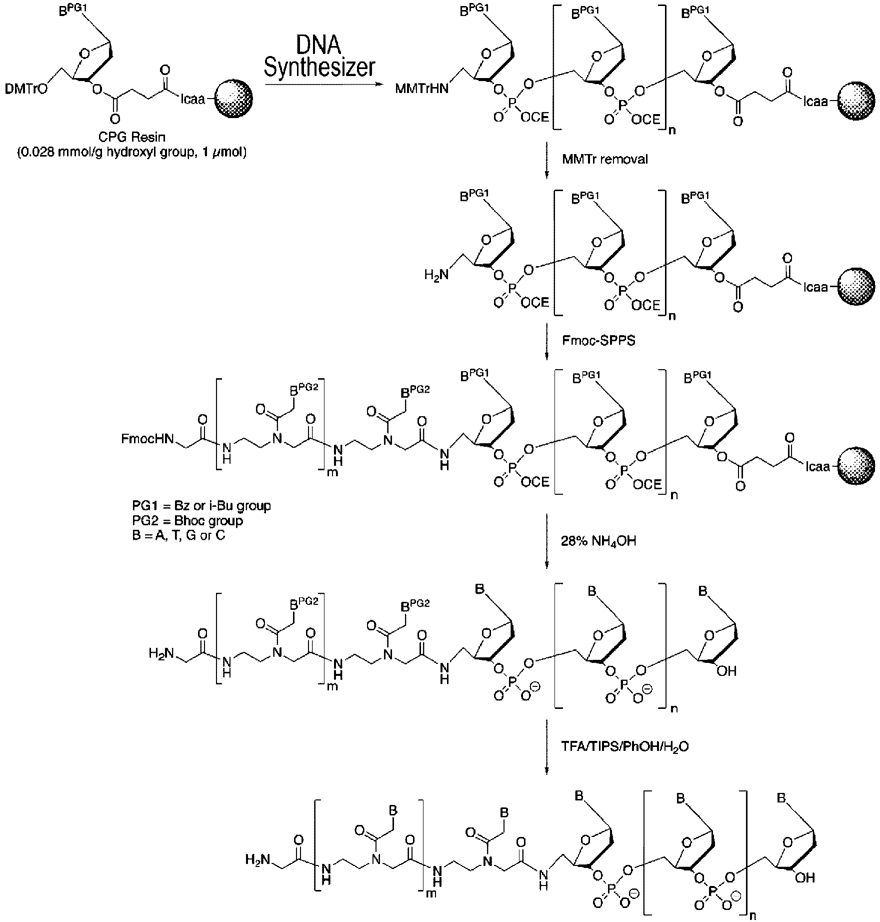

The N terminus of a PNA molecule is modified with glycine to suppress an acyl transfer reaction. The synthesis starts with the construction of a DNA molecule by an automated DNA/RNA synthesizer using a CPG resin bound to an adenine residue for DNA synthesis.

A DNA molecule can be extended by a conventional phosphoamidate method using 5-BMT as an activator. A 5'-amino-modified deoxynucleotide derivative is introduced into the 5' end of the DNA molecule using a 5'-MMTr (monomethoxytrityl) aminodeoxynucleotide phosphoamidate prepared by a known method. After the introduction of the 5'-amino-modified derivative by a DNA synthesizer, the MMTr group at the 5' end of the DNA is removed by performing a treatment with a 3% TCA/DCM solution for 15 minutes. Subsequently, in order to bind a PNA through an amide bond, it is extended by an Fmoc-SPPS method using a 5'-amino group of the DNA.

A condensation reaction using a benzoyl-protected FmocPNA is performed for 30 minutes with an NMP solution containing Fmoc-PNA(Bz)-OH (10 equivalents), HATU (10 equivalents), HOAt (10 equivalents), and DIEA (20 equivalents). The acetyl capping of an unreacted amino group on the resin can be carried out with 25% acetic anhydride/DCM. The Fmoc group can be removed by a treatment with 20% piperidine/NMP. The coupling, acetyl capping, and Fmoc removal steps are repeated until the target sequence is obtained. Finally, the dissociation and deprotection of a benzoyl group and a cyanoethyl group of the DNA molecule are carried out by performing a treatment with 28% ammonia for 60 minutes. The obtained crude product can be purified by reverse-phase HPLC, and the purified product can be identified by

### MALDI-TOF-MS.

### (Synthesis of PNA/PRNA-DNA Chimeric Molecule: P_{R}PD)

The synthesis of a P_{R}PD in which a PNA/PRNA is fused to the 5' side of a DNA can be carried out, for example, by the steps illustrated below in the same manner as in the synthesis of the PNA-DNA chimeric molecule described above.

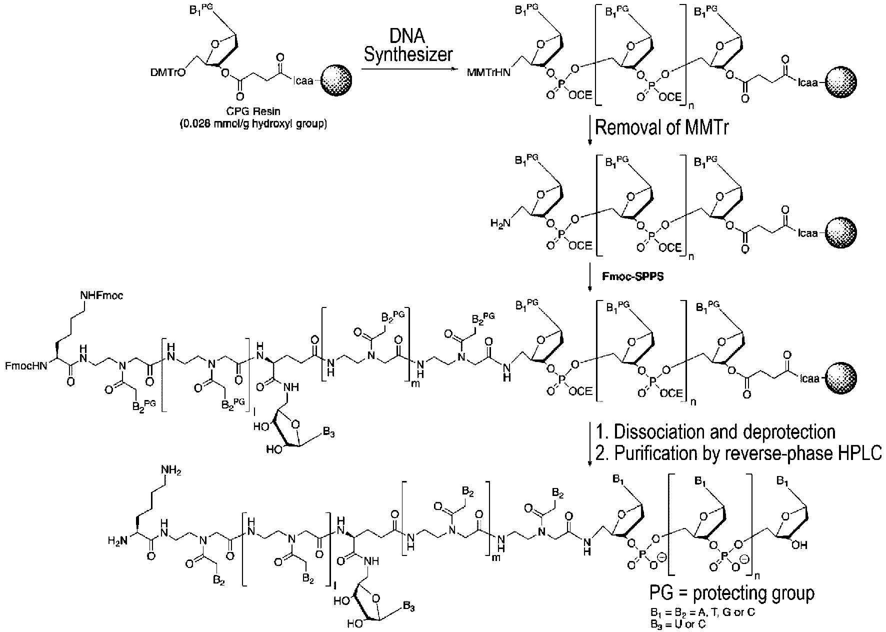

A RPNA monomer can be introduced into a specific site in a PNA molecule based on an Fmoc-SPPS method. The PRNA monomer (Fmoc-yPRNA-OH) can be prepared, for example, by the following steps.

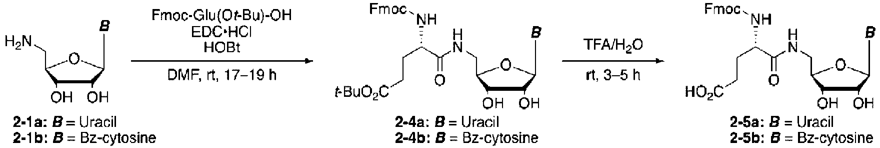

The crude product is purified by reverse-phase HPLC, and the purified product can be identified by MALDI-TOF-MS.

### (Synthesis of DNA-PNA/PRNA Chimeric Molecule: DP_{R}P)

The synthesis of a DP_{R}P in which a PNA/PRNA is fused to the 3' end of a DNA can be carried out, for example, by the following steps using an Fmoc-SPPS method with a DNA synthesizer.

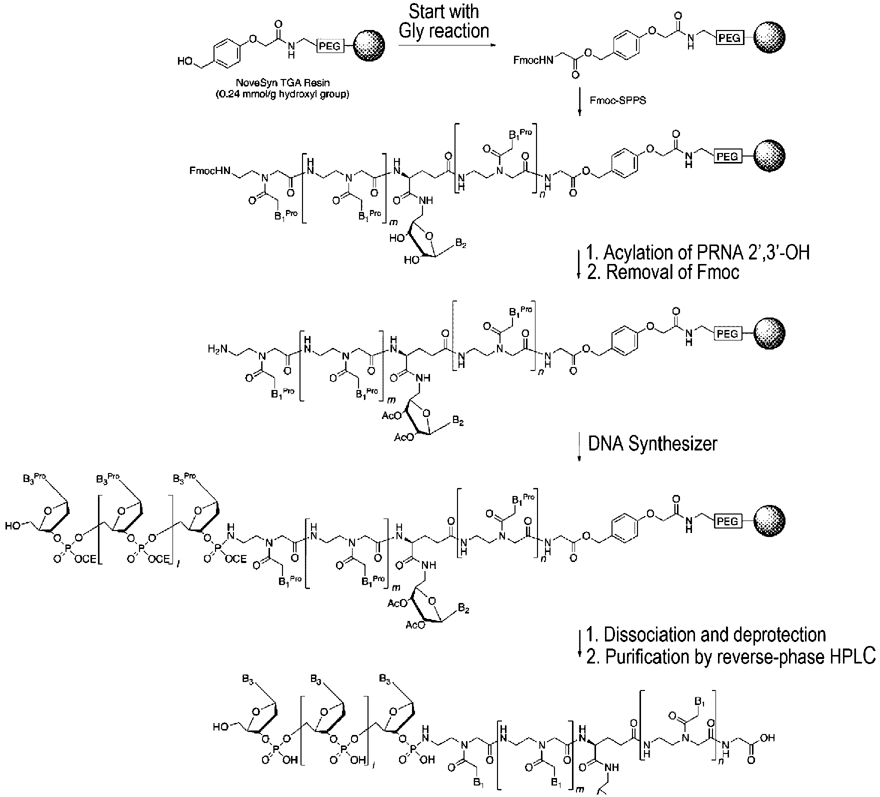

The synthesis starts with the introduction of Fmoc-Gly-OH on a NovaSyn (registered trademark) TGA resin using a condensing reagent of a DMF (N,N-dimethylformamide) solution of 1-(3-dimethylaminopropyl)-3-ethylarbodiimide hydrochloride (EDC·HCl) and 4-dimethylaminopyridine.

In the synthesis, it is preferred to use an Fmoc-Gly functional TGA resin for an Fmoc-SPPS method. A PNA/PRNA molecule can be synthesized by extension through an Fmoc-SPPS method using benzoyl-protected Fmoc-PNA and Fmoc-y PRNA monomers. As the coupling conditions, the same conditions as those for the PNA-DNA chimeric molecule can be used. After completion of Fmoc-SPPS, a 2',3'-hydroxy group of the PRNA molecule is protected with an acetyl group. After removing the N-terminal Fmoc group, the DNA molecule is extended from an N-terminal amino group of the PNA/PRNA molecule to which the resin is bound through a phosphoamidate bond by a known phosphoamidate method using a DNA synthesizer. Finally, dissociation and deprotection are performed with 28% ammonia. The obtained crude product can be purified by reverse-phase HPLC, and the purified product can be identified by MALDI-TOF-MS.

### (Action on Nuclease)

A complex composed of the chimeric molecule of the present invention and the target nucleic acid bound to the chimeric molecule specifically binds to a nuclease. As the nuclease, ribonuclease H is preferably used, but the nuclease is not limited thereto, and it need only specifically bind to the complex, and there is no particular restriction on the type of ribonuclease.

The nuclease has a site that recognizes the second nucleic acid or a derivative thereof (hereinafter, also referred to as a nucleic acid recognition site) and a nuclease active site that cleaves the target nucleic acid. The nucleic acid recognition site of the nuclease has a channel structure composed of a basic amino acid residue, and therefore, it binds to the second nucleic acid or a derivative thereof having an anionic skeleton. When the second nucleic acid or a derivative thereof binds to the nucleic acid recognition site, the first nucleic acid or a derivative thereof fused with the second nucleic acid or a derivative thereof is drawn to the cleavage active site of the nuclease, and the target nucleic acid can be selectively cleaved at the fusion (junction) site between the first nucleic acid or a derivative thereof and the second nucleic acid or a derivative thereof.

The chimeric molecule of the present invention can cleave the target sequence bound to the chimeric molecule of the present invention at the fusion (junction) site between the first nucleic acid or a derivative thereof and the second nucleic acid or a derivative thereof, and therefore, by designing a chimeric molecule in which the induction site is made to serve as a cleavage site in the target nucleic acid, the target nucleic acid can be cleaved at a target site.

In the chimeric molecule of the present invention, the melting temperature (Tm) of both fragments of the target nucleic acid after cleavage with the nuclease is preferably body temperature or lower, for example, 38°C or lower, or may be 37°C or lower, 30°C or lower, or 25°C or lower. By setting the Tm of both fragments of the target nucleic acid to body temperature or lower, both fragments of the target nucleic acid generated by cleavage at one site with the nuclease can be rapidly dissociated from the nuclease. On the other hand, the chimeric molecule of the present invention is rapidly dissociated from the nuclease after cleavage of the target nucleic acid with the nuclease, and therefore, the chimeric molecule of the present invention is rapidly used for cleavage of the subsequent target nucleic acid, and highly efficient turnover of the cleavage by the nuclease of the target nucleic acid can be achieved. Due to this, the chimeric molecule of the present invention can cleave the target nucleic acid at a low concentration, and thus, an off-target effect can be reduced.

### [Pharmaceutical Composition]

The pharmaceutical composition of the present invention contains the chimeric molecule of the present invention as an active ingredient.

In the present invention, the pharmaceutical composition is not particularly limited, but a pharmaceutical composition for treating a disease caused by a protein encoded by a target nucleic acid targeted by the chimeric molecule of the present invention or the like is exemplified, and examples thereof include an anti-tumor agent and a therapeutic agent for an ischemic brain disease. The pharmaceutical composition of the present invention can cleave a target nucleic acid by an endogenous nuclease and suppress the expression of a protein encoded by the target nucleic acid, and therefore can treat a disease caused by a protein encoded by the target nucleic acid.

As the protein encoded by the target nucleic acid, any protein can be adopted in principle as long as it becomes a target of the pharmaceutical composition of the present invention, and for example, TGF-β targeted for therapy for idiopathic pulmonary fibrosis, p53 or a cancer gene ras, each of which is a therapeutic target for cancer, VEGF 165 which is a therapeutic target for age-related macular degeneration, or the like is exemplified as an example of the protein targeted by the pharmaceutical composition of the present invention.

The disease is not particularly limited, and examples thereof include a cancer, an ischemic brain disease, age-related macular degeneration, familial hypercholesterolemia, muscular dystrophy, dementia, NASH, hepatic cirrhosis, idiopathic pulmonary fibrosis, a liver disease, an autoimmune disease, a renal disease, a hematopoietic disease, an atopic skin disease, and psoriasis.

The pharmaceutical composition of the present invention can be formulated into various forms, for example, a liquid (for example, an injection), a dispersion, a suspension, a tablet, a pill, a powder, a suppository, and the like. A preferred embodiment is an injection, which is preferably administered parenterally (for example, intravenously, transdermally, intraperitoneally, or intramuscularly).

As the pharmaceutical composition of the present invention, a most suitable dosage form or delivery system to be used for administration such as a combination agent with a nucleic acid drug having a different composition or a low molecular weight drug, an antibody drug, or the like may be selected in addition to being used as a single agent according to the above dosage form. Further, by using the pharmaceutical composition of the present invention as it is or by modifying it, various administration methods such as encapsulation in a micelle or a form bound to an antibody drug or the like can be selected. The pharmaceutical composition of the present invention varies depending on the type of disease, the type of target nucleic acid, or the like, but the dose can be set to, for example, 0.025 to 50 mg/kg, preferably 0.1 to 50 mg/kg, more preferably 0.1 to 25 mg/kg, and further more preferably 0.1 to 10 mg/kg or 0.1 to 3 mg/kg, but it is not limited thereto.

### [Method for Cleaving Target Nucleic Acid and Kit for Target Nucleic Acid Cleavage or Diagnosis]

The method for cleaving a target nucleic acid of the present invention uses the chimeric molecule of the present invention and a nuclease. Examples of the nuclease include those described above. A target sequence can be specifically cleaved by the method for cleaving a target nucleic acid of the present invention, and therefore, the method can be used for diagnosis of a disease caused by a protein encoded by the target nucleic acid other than for sequence selective cleavage of the target nucleic acid and for a genome editing tool.

The kit for target nucleic acid cleavage or diagnosis of the present invention includes the chimeric molecule of the present invention and a nuclease. Examples of the nuclease include those described above. The kit for target nucleic acid cleavage of the present invention can include other constituent elements necessary for detecting a target nucleic acid fragment, for example, an acrylamide gel, a reaction buffer solution, a reaction vessel, or the like in addition to the chimeric molecule of the present invention and the nuclease. A target nucleic acid can be specifically cleaved by the kit for target nucleic acid cleavage of the present invention, and therefore, the kit can be used for sequence selective cleavage of the target nucleic acid and as a genome editing tool. Further, the kit for diagnosis of the present invention can be used for diagnosis of a disease caused by a protein encoded by a target nucleic acid targeted by the chimeric molecule of the present invention. Examples of the protein encoded by the target nucleic acid targeted by the chimeric molecule of the present invention include those described above.

The kit for diagnosis of the present invention can, for example, determine whether or not a target nucleic acid is contained in a biological sample of a test subject by detecting whether or not a nucleic acid in the biological sample of the test subject can be cleaved by the kit for diagnosis of the present invention, and therefore, the test subject can be diagnosed whether the test subject has a disease caused by a protein encoded by the target nucleic acid targeted by the chimeric molecule of the present invention. The biological sample is not particularly limited, and examples thereof include blood, saliva, urine, cerebrospinal fluid, bone marrow fluid, pleural effusion, ascites, joint fluid, tear fluid, aqueous humor, vitreous fluid, and lymph fluid.

Further, by introducing the constituent elements of the kit for diagnosis of the present invention into the body, a structural change or a physicochemical change of a detection probe molecule caused by the internal body environment, the presence or absence of a target molecule, the concentration thereof, or the like can be detected outside the body, or an energy change due to light, magnetism, an ultrasonic wave, a radiation, or the like to be irradiated or exposed from the outside of the body can be measured with high sensitivity by external detection, and the kit can also be applied to in vitro diagnosis by visualization as an image.

### Examples

Next, the present invention will be described in more detail with reference to Examples, but the present invention is not limited to the following Examples.

### [Example 1] Production of PNA-DNA Chimeric Molecule

A PNA-DNA chimeric molecule was synthesized by automated DNA synthesis and an Fmoc-SPPS method using a CPG resin.

The synthesis was started with the construction of a DNA molecule using an automated DNA synthesizer. A CPG resin (1 µM scale) bound to an adenine residue for DNA synthesis, which is a CPG resin with a 2'-deoxyadenosine residue carried thereon, was introduced into the DNA synthesizer, and a DNA molecule was extended by a conventional phosphoamidate method using 5-BMT as an activator. The concentration of a phosphoamidate solution was set to 70 to 78 mM in aceronitrile. A 5'-amino-modified deoxycytidine derivative was introduced into the 5' end of the DNA molecule using 5'-MMTrNH deoxycytidine phosphoamidate prepared by a known method. After the introduction of the 5'-amino-modified derivative by the DNA synthesizer using the final trityl "ON" step, the resin was detached from the DNA synthesizer and transferred to a reaction column for an Fmoc-SPPS step. By performing a treatment with a 3% TCA/DCM solution for 15 minutes, the MMTr group at the 5' end of the DNA was removed.

Subsequently, a PNA was extended by an Fmoc-SPPS method using a 5'-amino group of the DNA as the base of an amide bond. The resin was treated with 20% piperidine/NMP to make a surface amino group basic, followed by filtration, and then, the resin was washed 5 or more times with NMP. A condensation reaction using a benzoyl group-protected Fmoc PNA monomer was performed by a treatment using an NMP solution (300 µL) containing Fmoc-PNA(Bz)-OH (10 equivalents), HATU (10 equivalents), HOAt (10 equivalents), and DIEA (20 equivalents). After the reaction was allowed to proceed at room temperature for 30 minutes with occasional vigorous stirring, the solution was removed and the resin was washed 3 or more times with NMP.

Subsequently, a 20% piperidine solution in NMP was added to the resin and stirred occasionally for 20 minutes to remove an Fmoc group. After the Fmoc group removal was completed, the resin was washed 5 or more times with NMP. The condensation reaction and the Fmoc removal step were repeated until the sequence of the target PNA-DNA chimeric molecule was constructed.

Finally, in order to suppress the molecular acyl transfer in the N-terminal amino group of the PNA residue, Fmoc-Gly-OH was introduced under the same condensation conditions as described above. After the Fmoc-SPPS step was completed, the resin was treated with a 28% ammonia solution at 60°C for 16 hours to remove all protecting groups and separate them from the resin. Subsequently, the resin was removed by filtration and the filtrate was concentrated. The crude product was purified using reverse-phase HPLC under the conditions of a linear gradient from 5% to 70% with a solvent A: a 0.1 M TEAA buffer solution (pH 7.5) and a solvent B: 0.1 M TEAA (pH 7.5)/acetonitrile (1:1, v/v) at a flow rate of 3 mL/min for 30 minutes using a COSMOSIL 5C₁₈-AR-II column (Nacalai Tesque, Φ 10 × 250 mm).

By the above-mentioned method, two types of PNA-DNA chimeric molecules (PD1 and PD2) shown in the following Table 1 were produced.

**[Table 1]**

| Chimeric molecule | Base sequence | SEQ ID NO | Type of chimeric molecule | Length |
|---|---|---|---|---|
| PD1 | N-Gly-(gtatatct)-(^{HN}CCTTCTTA)-3' | 1 | PNA-DNA chimera | 16 mer |
| PD2 | N-Lys-(tgcaagacta)-(^{HN}TAAGATTC)-3' | 2 | PNA-DNA chimera | 18 mer |

A lower case letter denotes a PNA base, an upper case letter denotes a DNA base, and ^{HN}T and ^{HN}C each denote a 5'-amino substituted DNA base.

### [Example 2] Production of P_{R}PD

A P_{R}PD which is a DNA-PNA/PRNA chimera resulting from fusion of a PNA/PRNA to the 5'-end side of a DNA was synthesized by a combination of an automated DNA synthesizer and an Fmoc step using a CPG resin.

The synthesis was started with the construction of a DNA molecule by automated DNA synthesis. A CPG resin (1 µM scale) that carries a 2'-deoxynucleoside residue was introduced into a DNA synthesizer, and a DNA molecule was extended by a conventional phosphoamidate method using 5-BMT as an activator. The concentration of a phosphoamidate solution was set to 70 to 78 mM in aceronitrile. A 5'-amino-modified deoxynucleoside derivative was introduced into the 5' end of the DNA molecule using 5'-MMTrNH deoxycytidine phosphoamidate prepared by a known method.

After the introduction of the 5'-amino-modified derivative by the DNA synthesizer using the final trityl "ON" step, the resin was detached from the DNA synthesizer and transferred to a reaction column for an Fmoc-SPPS step. By performing a treatment with a 3% TCA/DCM solution for 15 minutes, the MMTr group at the 5' end of the DNA was removed.

Subsequently, a PNA was extended by an Fmoc-SPPS method using a 5'-amino group of the DNA as the base of an amide bond. The resin was treated with 20% piperidine/NMP to make a surface amino group basic, followed by filtration, and then, the resin was washed 5 or more times with NMP.

A condensation reaction using a benzoyl group-protected Fmoc PNA monomer was performed by a treatment using an NMP solution (300 µL) containing Fmoc-PNA(Bz)-OH (10 equivalents), HATU (10 equivalents), HOAt (10 equivalents), and DIEA (20 equivalents). After the reaction was allowed to proceed at room temperature for 30 minutes with occasional vigorous stirring, the solution was removed and the resin was washed 3 or more times with NMP.

Subsequently, a 20% piperidine solution in NMP was added to the resin and stirred occasionally for 20 minutes to remove an Fmoc group. After the Fmoc group removal was completed, the resin was washed 5 or more times with NMP. The condensation reaction and the Fmoc removal step were repeated until the sequence of the target PNA-DNA chimeric molecule was constructed. Finally, in order to suppress the molecular acyl transfer in the N-terminal amino group of the PNA residue, Fmoc-Gly-OH or Fmoc-Lys(Fmoc)-OH was introduced under the same condensation conditions as described above.

After the Fmoc-SPPS step was completed, the resin was treated with a 28% ammonia solution at 60°C for 16 hours to remove all protecting groups and separate them from the resin.

Subsequently, the resin was removed by filtration and the filtrate was concentrated. The crude product was purified using reverse-phase HPLC under the conditions of a linear gradient from 5% to 70% with a solvent A: a 0.1 M TEAA buffer solution (pH 7.5) and a solvent B: 0.1 M TEAA (pH 7.5)/acetonitrile (1:1, v/v) at a flow rate of 3 mL/min for 30 minutes using a COSMOSIL 5C₁₈-AR-II column (Nacalai Tesque, Φ 10 × 250 mm).

By the above-mentioned method, seven types of P_{R}PDs (P_{R}PD1 to P_{R}PD7) shown in Table 2 were produced.

**[Table 2]**

| Chimeric molecule | Base sequence | SEQ ID NO | Type of chimeric molecule | Length |
|---|---|---|---|---|
| P_{R}PD1 | N-Lys-(tgcaagac*U*a)-(^{HN}TAAGATTC)-3' | 3 | PRNA-PNA-DNA chimera | 18 mer |
| P_{R}PD2 | N-Lys-(gta*U*atctcc)-(^{HN}TTCTTA)-3' | 4 | PRNA-PNA-DNA chimera | 16 mer |
| P_{R}PD3 | N-Lys-(aag*U*act*U*ag)-(^{HN}CGTAAG)-3' | 5 | PRNA-PNA-DNA chimera | 16 mer |
| P_{R}PD4 | N-Lys-(tgCaagacta)-(^{HN}TAAGATTC)-3' | 6 | PRNA-PNA-DNA chimera | 18 mer |
| P_{R}PD5 | N-Gly-(tgcaagac*C*ta)-(^{HN}TAAGATTC)-3' | 7 | PRNA-PNA-DNA chimera | 18 mer |
| P_{R}PD6 | N-Lys-(tgcaagac*C*ta)-(^{HN}TAAGATTCAAT)-3' | 8 | PRNA-PNA-DNA chimera | 21 mer |
| P_{R}PD7 | N-Gly-(gta*U*atct)-(^{HN}CCTTCTTA)-3' | 9 | PRNA-PNA-DNA chimera | 16 mer |

An italic letter denotes a PRNA base, a lower case letter denotes a PNA base, an upper case letter denotes a DNA base, and ^{NH}T and ^{NH}C each denote a 5'-amino substituted DNA base.

### [Example 3] Production of DP_{R}P

Fmoc-Gly-OH (30 mg, 100 µmol) and EDC·HCl (19 mg, 100 µmol) were dissolved in DMF (1.0 mL), and the solution was stirred under ice cooling for 35 minutes. Ice cooling was removed, and a NovaSyn (registered trademark) TGA resin (42 mg, 10 µmol of hydroxy group) in DMF and DMAP (1.2 mg, 10 µmol) was added to a solution of DMF and DMAP (1.2 mg, 10 µmol), and the resulting mixture was stirred at room temperature for 1.5 hours. The resin was filtered and washed continuously with NMP, DCM/ethanol (1:1, v/v) and DCM.

After the resin was thoroughly washed, 1.0 mL of a 20% pyridine/NMP solution of benzoic anhydride (113 mg, 0.5 mmol) was added to the resin, and the mixture was left at room temperature with occasional shaking. After leaving it for 1 hour, the resin was thoroughly washed with NMP and DCM. After washing with ethanol, the resin was dried under reduced pressure in a desiccator, whereby an Fmoc-Gly functional TGA resin was obtained (41 mg, 94%). The introduction amount of Fmoc-Gly-OH was calculated by quantitative UV (301 nm) measurement of Fmoc-piperidine released by a treatment with 20% piperidine/NMP.

The Fmoc-Gly functional TGA resin (1 µmol scale) obtained above was washed with NMP and treated with 20% piperidine/NMP at room temperature for 20 minutes to remove an Fmoc protecting group. After thorough washing with NMP, the resin was added to an NMP solution (300 µL) of Fmoc-PNA(Bz)-OH or Fmoc-yRPNA-OH (10 equivalents), HOAt (10 equivalents), HATU (10 equivalents), and DIEA (20 equivalents), and the mixture was left at room temperature for 30 minutes with occasional shaking, and this coupling step was repeated twice. The resin was washed with NMP and then washed with DCM, and thereafter, the resin was treated with 25% acetic anhydride/DCM at room temperature for 10 minutes. After the coupling step, the resin was washed with DCM and then washed with NMP. Subsequently, Fmoc was removed by a treatment with 20% piperidine/NMP at room temperature for 20 minutes. The same Fmoc removal, coupling, and capping steps were used to extend a PNA-PRNA strand. After the final coupling step, the resin was thoroughly washed with NMP and DCM and then washed with pyridine. Thereafter, acetic anhydride/DCM (2:3, v/v) was added to the resin and left at room temperature for 2 hours with occasional shaking, whereby a 2',3'-hydroxy group of the PRNA molecule was protected.

After the N-terminal Fmoc protecting group was removed, the resin was transferred to an empty column for an automated DNA/RNA synthesizer and the column containing the resin was placed in the DNA/RNA synthesizer. The DNA strand was extended by a conventional phosphoamidate method using a 5'-O-DMTr-3'-O-(2-cyanoethyl)phosphoamidate construction block. After the strands were collected, the resin was treated with a 28% aqueous ammonia solution at 60°C for 18 hours to deprotect and dissociate a DP_{R}P. Thereafter, the resultant was filtered through a membrane filter to remove the resin. The filtrate was partially concentrated in vacuo and purified using reverse-phase HPLC with a 5C₁₈-AR-II column (10 × 250 nm), whereby a DP_{R}P was obtained.

By the above-mentioned method, two types of DP_{R}Ps (DP_{R}P1 and DP_{R}P2) shown in Table 3 were produced.

**[Table 3]**

| Chimeric molecule | Base sequence | SEQ ID NO | Type of chimeric molecule | Length |
|---|---|---|---|---|
| DP_{R}P1 | 5'-(TGCAAGAC)-(ta*U*aagattc)-Gly-C | 10 | DNA-PRNA-PNA chimera | 18 mer |
| DP_{R}P2 | 5'-(GTATATCT)-(ccttc*U*ta)- Gly-C | 11 | DNA-PRNA-PNA chimera | 16 mer |

An italic letter denotes a PRNA base, a lower case letter denotes a PNA base, an upper case letter denotes a DNA base, and ^{HN}T and ^{HN}C each denote a 5'-amino substituted DNA base.

### [Example 4] Measurement of RNase Activity

The RNA cleavage activity by an RNase of a P_{R}PD·RNA complex was examined as follows.

A 5'-FAM labeled RNA (5'-FAM-GAAUCUUAUAGUCUUGCA-3': RNA1) in which a target base sequence has the base sequence represented by SEQ ID NO: 12 was mixed with 0.1 equivalents of P_{R}PD1 obtained in Example 2. The mixture was allowed to anneal to form a P_{R}PD·RNA complex.

Subsequently, 60 U/µL of RNase H was added to the mixture and allowed to react therewith at 37°C to cleave RNA1. After the reaction was allowed to proceed for 30 minutes, a 7 M urate tris hydrochloride buffer solution was added thereto to stop the reaction, and subsequently, the RNase H was inactivated at 70°C for 10 minutes. As a control of P_{R}PD1, the same treatment was performed for DNA1 (5'-TGCAAGACTATAAGATTC-3') having the base sequence represented by SEQ ID NO: 13 of a counterpart. The cleaved RNA fragments were subjected to 20% degradation polyacrylamide gel electrophoresis (degradation PAGE) and analyzed by visualization with a fluorescent image analyzer and quantitative determination. The results are shown in FIG. 1 and Table 4.

**[Table 4]**

| Substrate | RNase H (U/µL) | |
|---|---|---|
| | 60 | 6 |
| DNA1·RNA1 | 54% | 12% |
| P_{R}PD1·RNA1 | >99% | 96% |

As shown in FIG. 1 and Table 4, in the case of DNA1 using 60 U/µL of RNase H, 54% of RNA1 was cleaved. On the other hand, in the case of P_{R}PD1, 99% or more of RNA1 was cleaved, which means saturation of the activity of RNase H. Therefore, the same RNA cleavage experiment was performed by reducing the amount of RNase H to 6 U/µL.

As a result, as shown in FIG. 1 and Table 4, in the case of DNA1, the extent of cleavage of RNA1 decreased to 12%, whereas in the case of P_{R}PD1, the cleavage of RNA1 was maintained at 90% or more. As a result, it was revealed that as compared with the DNA1·RNA1 complex, RNase H exhibited an 8 times higher RNA1 cleavage activity in the P_{R}PD1·RNA1 complex.

The cleavage site in the case of P_{R}PD1 was one site near the PNA-DNA amide bond as shown in FIG. 2B. On the other hand, in the case of DNA1, cleavage occurred at multiple sites as shown in FIG. 2A. The minimum base length of a DNA to which RNase H binds is 5 to 6 bases, which corresponds to a distance between the DNA binding channels of RNase H. Therefore, the DNA molecule of P_{R}PD1 was composed of 7 DNA nucleobases which are accurately recognized by the DNA binding channel of RNase H to introduce one cleavage site. A DNA·RNA heterocomplex using 5 to 6 base pairs enables binding at several sites. This induces RNA cleavage at multiple sites. Based on these, the enhancement of the above RNA cleavage is explained as follows.

RNase H does not have base sequence selectivity for a target RNA, and therefore, in the case of DNA1, there are various cleavage sites in the complex with the target RNA, and it is highly possible that one cleavage does not induce dissociation of the complex, and from an ASO-cleaved RNA-RNase H complex, the cleaved RNA cannot be dissociated, and the turnover number of DNA1 is low. On the other hand, it is considered that in the case of P_{R}PD1 which is the chimeric molecule of the present invention, cleavage at one site in the target RNA, particularly before and after the sequence at which the stability of the complex after cleavage becomes at body temperature (37°C) or lower in any case can be achieved, and the complex is efficiently dissociated by one cleavage, and it can bind to another target RNA that is not cleaved, and the turnover number can be dramatically improved. This was demonstrated by calculating the Tm of a complex of an ASO and two fragments of the cleaved RNA1·DNA1 complex after cleavage by actual measurement or the nearest neighbor method using a thermodynamic parameter.

As shown in FIG. 2A, in the case of DNA1, cleavage was observed nonspecifically at various sites (a to e). When the stability of the complex after cleavage was calculated, in the case of cleavage at the site a and the site d, the post-cleavage complexes with DNA1 had a Tm of 35.3°C and 37.2°C, respectively, each of which is almost the same as the body temperature (37°C), and it is expected that the complex is less likely to dissociate even after cleavage. Also in the case of cleavage at the other sites, many of the complexes with the cleavage fragment had a Tm of 37°C or higher. In the case of some of the cleavage fragments (site b or c), some complexes of the RNA fragment after cleavage and DNA1 had a Tm of 37°C or lower, but the probability of generation thereof was not high. For this reason, RNase H cleavage is generally needed multiple times for complex dissociation, and suppression of the turnover number of RNase H cleavage occurs.

On the other hand, P_{R}PD1 showed selective one RNA cleavage at a PNA-DNA junction, which greatly reduces the temperature stability of the RNA1·P_{R}PD1 complex as compared with that before RNA cleavage. Therefore, a single RNase H cleavage dissociates the complex so as to enable an ASO to immediately form a complex with an uncleaved target RNA, and as a result, the turnover number of RNase H cleavage is enhanced, and all RNA1 fragments are cleaved in this experiment. In order to confirm this, the Tm of the cleaved RNA·P_{R}PD1 complex was calculated.

As shown in FIG. 2B, the major cleavage site was one site: a site a. Therefore, a generated fragment forms a complex of a type different from a complex with P_{R}PD1 such as a complex with the DNA molecule of P_{R}PD1 (cleaved RNA·DNA complex) or a complex with the PNA/PRNA molecule of P_{R}PD1 (cleaved RNA·PNA/PRNA complex). When the temperature stability of the cleaved RNA·DNA complex was calculated using the nearest neighbor thermodynamic parameters, the Tm was 13.2°C. As for the temperature stability of the cleaved RNA·PNA/PRNA complex, the Tm was 22.3°C by a UV temperature melting analysis. It has been reported that a PNA forms an unstable complex with a DNA/RNA under physiologically high salt concentration conditions. Due to this, the low temperature stability of the cleaved RNA·PNA/PRNA complex is considered to be rational.

From the above, the cleavage of RNA1 at selective one site in the PNA-DNA fusion part forms an RNA fragment having a temperature stability of the body temperature (37°C) or lower, and is recognized as an efficient turnover of P_{R}PD1.

Subsequently, the effect of introduction of a PRNA into a chimera on the RNase H activity was examined. RNA1 (1 µM) and RNase H (0.6 U/µL) were allowed to react at 37°C for 30 minutes in the presence of DNA1, P_{R}PD4, P_{R}PD1, or PD2 (10 nM). The results are shown in FIG. 3 and Table 5.

**[Table 5]**

| Substrate | [DNA1], [P_{R}PD4], [P_{R}PD1], or [PD2]/ nM |
|---|---|
| | 10.0 |
| DNA1·RNA1 | 9% |
| P_{R}PD4·RNA1 | 95% |
| P_{R}PD1·RNA1 | 96% |
| PD2·RNA1 | 93% |

In the case of DNA1, the amount of RNA1 cleavage was very small. On the other hand, cleavage of more than 90% of RNA1 was observed in the presence of P_{R}PD4 and P_{R}PD1 at 10 nM. Even in the case of PD2, the RNase H activity at the same level as compared with the case of P_{R}PD4 and P_{R}PD1 was exhibited.

The effect of introduction of a PRNA into a chimera on the RNase H activity was examined with a 16-mer RNA using RNA2 (5'-FAM-UAAGAAGGAGAUAUAUC-3') having the base sequence represented by SEQ ID NO: 14. RNA2 (1 µM) and RNase H (6 × 10³ U/µL) were allowed to react at 37°C for 30 minutes in the presence of DNA2 (5'-GTATATCTCCTTCTTA-3') having the base sequence represented by SEQ ID NO: 15, P_{R}PD7, or PD1 (10 nM). The results are shown in FIG. 4 and Table 6.

**[Table 6]**

| Substrate | [DNA2], [P_{R}PD7], or [PD1]/ nM |
|---|---|
| | 10 |
| DNA2·RNA2 | 52% |
| P_{R}PD7·RNA2 | 76% |
| PD1·RNA2 | 40% |

In the case of DNA2, 52% of RNA2 was cleaved. In the presence of P_{R}PD7, cleavage of 76% of RNA2 was observed, which was 1.5 times the RNase H activity as compared with the case of DNA2. On the other hand, in the case of PD1, the amount of RNA2 cleavage was 40%, which was 1.9 times lower than in the case of P_{R}PD7. As a result, the introduction of a PRNA into the chimeric molecule makes it possible to enhance the RNase H activity, which is considered to be due to RNA-selective binding affinity to form a type A complex with the target RNA.

Subsequently, the target RNA1 cleavage activity by RNase H of DP_{R}P1 was examined and compared with the activity of P_{R}PD5. The DP_{R}P is expected to induce RNA cleavage at the 3' end. RNA1 was mixed with 1/100, 1/1000, or 1/10000 equivalents of DP_{R}P1 or P_{R}PD5, and the mixture was allowed to anneal to form a complex. Thereafter, 6 × 10³ U/µL of RNase H was added thereto and allowed to react at 37°C for 30 minutes. The results are shown in FIG. 5 and Table 7.

**[Table 7]**

| Substrate | [DP_{R}P1], or [P_{R}PD5]/ nM | | |
|---|---|---|---|
| | 10.0 | 1.0 | 0.1 |
| DP_{R}P1·RNA1 | 43% | 2% | <1% |
| P_{R}PD5·RNA1 | 73% | 12% | >1% |

As shown in FIG. 5, DP_{R}P1 cleaved the RNA at a site 3 to 4 bases from the 3' end of the RNA. The major cleavage sites are shown in FIG. 6. The cleavage activity was 43% in the case of 10 nM DP_{R}P1, which was 1.7 times lower than in the case of DP_{R}P5 (73%). The low cleavage activity of DP_{R}P1 is considered to be due to the limiting RNA1 cleavage site. In the case of P_{R}PD5, the cleavage site is one point at the PNA-DNA junction. Therefore, efficient dissociation of RNA1 and efficient turnover of P_{R}PD5 were achieved by the formation of RNA1 having a low temperature stability as low as 37°C or lower.

On the other hand, in the case of DP_{R}P1, cleavage was observed near the 3' end of RNA1, and therefore, a long RNA fragment was generated after the cleavage, which maintains a high temperature stability above 37°C. Therefore, the turnover efficiency of DP_{R}P1 decreased as compared with P_{R}PD5.

### [Example 5] Kinetic Parameters of RNA Cleavage Reaction by RNase H

In order to examine the highly efficient RNA cleavage of P_{R}PD5, a kinetic analysis of RNA cleavage by RNase H was performed.

RNase H (1.5 × 10³ U/µL = 4.1 nM) was allowed to react in the presence of an excess amount of P_{R}PD1 RNA1 (1, 2, 5, or 10 µM) under pseudo-first-order reaction conditions, and a time profile of the amount of RNA1 cleaved at each P_{R}PD1·RNA1 concentration was plotted. By using the obtained initial rate at each substrate concentration, a Lineweaver-Burk plot was made. The same experiment was performed also for the case of DP_{R}P1·RNA1. The kinetic parameters obtained from the Lineweaver-Burk plot was summarized in Table 8. The parameters of a DNA/RNA complex were extracted with reference to the results reported by Otsuka et al. The results are shown in Table 8.

**[Table 8]**

| | K_{cat}/ s⁻¹ | Kₘ/ ×10⁻⁶ M | Vₘₐₓ/ ×10⁻⁸ Ms⁻¹ | k_{cat}/Kₘ/ ×10⁶ s⁻¹M⁻¹ |
|---|---|---|---|---|
| DNA·RNA | 8.3 | 0.67 | 1.6 | 12.4 |
| P_{R}PD1·RNA1 | 16 | 9.6 | 6.7 | 1.67 |
| DP_{R}P1·RNA1 | 6.9 | 3.5 | 2.8 | 1.97 |

As shown in Table 8, the k_{cat} value of the RNA cleavage reaction by the P_{R}PD1·RNA1 complex and RNase H was about 1.9 times that in the case of the DNA·RNA complex. This result indicates that the cleavage efficiency of RNase H is slightly improved in the case of the P_{R}PD1·RNA1 complex as compared with the case of the corresponding DNA·RNA complex, however, the improvement ratio is clearly lower than in the target RNA cleavage experiment shown in FIG. 3. RNase H exhibited a high cleavage activity with the P_{R}PD1·RNA1 complex, and the Michaelis-Menten multiplier (Km) of binding of RNase H to the P_{R}PD1·RNA1 complex was higher than in the case of the DNA·RNA complex. This suggests that the binding activity of the P_{R}PD1·RNA1 complex to RNase H is lower than in the case of the DNA·RNA complex. According to the relationship between the k_{cat} and Km values, it is considered that the recognition property of RNase H hardly affects the high RNA cleavage activity of P_{R}PD1. Further, the Vₘₐₓ value of the P_{R}PD1·RNA1 complex is 4 times larger than that of the DNA·RNA complex, but this value is smaller than a value estimated from gel electrophoresis (for example, from FIG. 1, an about 8 times higher RNA cleavage activity was observed (comparison between Lanes 2 and 4)). From these results, it is considered that the binding of RNase H to the substrate (P_{R}PD1-RNA1 complex) is not a rate-limiting step, and the step of dissociation of the cleaved P_{R}PD1-RNA1 complex from RNase H is important for the efficient RNA cleavage activity of RNase H.

In the case of DP_{R}P1, the k_{cat} value of the RNA cleavage activity by RNase H was smaller than in the case of the DNA·RNA complex. This result indicates that DP_{R}P1 shows a lower cleavage activity than the corresponding DNA. The Vₘₐₓ value of RNA cleavage was 1.7 times or more higher than in the case of the DNA·RNA complex, but the Km value was 5.2 times or more higher than in the case of the DNA·RNA complex.

The Km value higher than in the case of the DNA·RNA complex is considered to be due to the fact that a decrease in the recognition property of RNase H is induced by substituting a natural DNA skeleton with a PNA-PRNA skeleton. However, the Km value of the DP_{R}P1·RNA1 complex is lower than that of P_{R}PD1-RNA1, which indicates that the recognition property of RNase H for the DP_{R}P1·RNA1 complex is enhanced as compared with the case of the P_{R}PD1·RNA1 complex. The fact that the Km value is different between DP_{R}P1·RNA1 and P_{R}PD1·RNA1 supports the fact that the recognition site by RNase H in the DP_{R}P1·RNA1 complex is different from the recognition site in the P_{R}PD1-RNA1 complex.

The cleavage reaction shown in FIG. 5 also supports the fact that the recognition sites are different. The RNase H activity different between the P_{R}PD and the DP_{R}P is considered to be dependent not only on the RNA cleavage site, but also on the recognition property of RNase H and the catalytic cleavage activity of RNase H (k_{cat}/Kₘ). The k_{cat}/Kₘ value can be determined by the substrate concentration dependent on the kinetic analysis of the RNA cleavage reaction of RNase H. As shown in the above Table 8, the k_{cat}/Kₘ values of the P_{R}PD and the DP_{R}P were in substantially the same order (P_{R}PD·RNA: 1.67 × 10⁶ s⁻¹M⁻¹, DP_{R}P·RNA: 1.97 × 10⁶ s⁻¹M⁻¹). Based on this, it is considered that the turnover efficiency of the chimeric molecule is controlled mainly by the cleavage site in the RNA. These revealed that the cleavage site of RNase H is important for enhancing the RNA cleavage activity and the turnover efficiency of the chimera.

### [Example 6] Antisense Activity in Cell-Free Translation System

In order to confirm the application to endogenous RNA cleavage, by using an in vitro cell-free translation system, the protein synthesis inhibitory activity (antisense activity) of a P_{R}PD was examined. P_{R}PD2 and DNA2, each of which is an ASO used in this examination, are 16-mer fragments containing 5'-GAAGGA-3' which is a complementary sequence of the Shine-Dalgarno (SD) sequence upstream of a Renilla luciferase gene for a luciferase reporter assay. The ASO (P_{R}PD2 or DNA2) was added in an amount of 1/10 equivalents with respect to a target mRNA. The reaction was allowed to proceed in the presence and absence of RNase H in order to evaluate the RNA cleavage activity of RNase H. The results are shown in FIG. 7.

As shown in FIG. 7, in the absence of RNase H, the antisense activity was almost not observed in the case of DNA2. The inhibitory activity of DNA2 was slightly observed by the addition of RNase H (12%). On the other hand, in the case of P_{R}PD2, a 9% antisense activity was observed in the absence of RNase H, which was slightly higher than in the case of DNA1. The antisense activity of P_{R}PD2 was remarkably enhanced to 91% by the addition of RNase H. Such a low level lower than the stoichiometric inhibition level in the absence of RNase H indicates that inhibition occurs only by the formation of a one-to-one complex. By the addition of only 1/10 equivalents of an antisense oligonucleotide, in the case of P_{R}PD2, 10% or more inhibition of protein expression was observed. This indicates that P_{R}PD2 acts like a catalyst by the RNase H activity. This result suggests that the P_{R}PD can inhibit protein production by efficiently cleaving a target mRNA and utilizing the RNase H activity. This result matches with the gel electrophoresis analysis of the RNA fragments. Accordingly, the examination of the RNA cleavage site is considered essential to design a more effective ASO for the antisense activity.

Subsequently, the antisense activity of each of P_{R}PD5 and DP_{R}P2 in a cell-free translation system was examined using PURSYSTEM Classic II. The experiment was performed in the absence and presence of RNase H. The results are shown in FIG. 8.

Each of P_{R}PD5 and DP_{R}P2 was added in an amount of 1/10 equivalents with respect to a target mRNA encoding a Renilla luciferase gene. In the case of P_{R}PD5, the antisense activity was enhanced to 75% by the addition of RNase H. On the other hand, in the case of DP_{R}P2, the antisense activity was enhanced to 12% by the addition of RNase H, which was 6 times lower than in the case of P_{R}PD5.

### Industrial Applicability

According to the present invention, a chimeric molecule capable of restraining the function of a target nucleic acid at a low concentration and suppressing an off-target effect, a pharmaceutical composition containing the chimeric molecule, a method for cleaving a target nucleic acid using the chimeric molecule, and a kit for target nucleic acid cleavage or diagnosis containing the chimeric molecule can be provided.

## Claims

1. A chimeric molecule resulting from fusion of a first nucleic acid or a derivative thereof, which has an ability to bind to a target nucleic acid, with a second nucleic acid or a derivative thereof, which has an ability to bind to the target nucleic acid, and in which a main chain skeleton is anionic.

2. The chimeric molecule according to claim 1, wherein a main chain skeleton of the first nucleic acid or a derivative thereof is neutral or cationic.

3. The chimeric molecule according to claim 2, wherein the main chain skeleton of the first nucleic acid or a derivative thereof is an amide skeleton.

4. The chimeric molecule according to any one of claims 1 to 3, wherein the main chain skeleton of the second nucleic acid or a derivative thereof is a sugar-phosphate skeleton.

5. The chimeric molecule according to any one of claims 1 to 4, wherein the first nucleic acid or a derivative thereof is fused to the 5' end of the second nucleic acid or a derivative thereof.

6. The chimeric molecule according to any one of claims 1 to 4, wherein the first nucleic acid or a derivative thereof is fused to the 3' end of the second nucleic acid or a derivative thereof.

7. The chimeric molecule according to any one of claims 1 to 6, wherein a complex composed of the chimeric molecule and the target nucleic acid bound to the chimeric molecule specifically binds to a nuclease.

8. The chimeric molecule according to claim 7, wherein the nuclease cleaves the target nucleic acid at a fusion part of the first nucleic acid or a derivative thereof and the second nucleic acid or a derivative thereof.

9. The chimeric molecule according to claim 8, wherein the melting temperature Tm of both fragments of the target nucleic acid after cleavage with the nuclease is 38°C or lower.

10. The chimeric molecule according to any one of claims 7 to 9, wherein the nuclease is ribonuclease H.

11. The chimeric molecule according to any one of claims 1 to 10, wherein the first nucleic acid or a derivative thereof is a peptide nucleic acid or a peptide ribonucleic acid or a derivative thereof.

12. The chimeric molecule according to any one of claims 1 to 11, wherein the second nucleic acid or a derivative thereof is a DNA.

13. The chimeric molecule according to any one of claims 1 to 12, wherein the target nucleic acid is an RNA or a DNA.

14. A pharmaceutical composition, comprising the chimeric molecule according to any one of claims 1 to 13 as an active ingredient.

15. The pharmaceutical composition according to claim 14, wherein the pharmaceutical composition is a cancer or an ischemic brain disease.

16. A method for cleaving a target nucleic acid, comprising cleaving a target nucleic acid using the chimeric molecule according to any one of claims 1 to 13 and a nuclease.

17. The method for cleaving a target nucleic acid according to claim 16, wherein the nuclease is ribonuclease H.

18. A kit for target nucleic acid cleavage or diagnosis, comprising the chimeric molecule according to any one of claims 1 to 13 and a nuclease.

19. The kit according to claim 18, wherein the nuclease is ribonuclease H.


SEQUENCE LISTING

<110>  Tohoku university

<120>  Chimera molecule, pharmaceutical composition, cleavage method of
       target nucleic acid, and kit for cleaving target nucleic acid or
       for diagnosis

<130>  PC-30427

<140>  JP 2019-136414
<141>  2019-07-24

<160>  15

<170>  PatentIn version 3.5

<210>  1
<211>  16
<212>  DNA
<213>  Artificial Sequence

<220>
<223>  PNA-DNA chimera


<220>
<221>  misc_feature
<222>  (1)..(8)
<223>  peptide nucleic acid (PNA)

<220>
<221>  misc_feature
<222>  (9)..(9)
<223>  5'-amino-substituted DNA

<400>  1
gtatatctcc ttctta                                                    16


<210>  2
<211>  18
<212>  DNA
<213>  Artificial Sequence

<220>
<223>  PNA-DNA chimera


<220>
<221>  misc_feature
<222>  (1)..(10)
<223>  peptide nucleic acid (PNA)

<220>
<221>  misc_feature
<222>  (11)..(11)
<223>  5'-amino-substituted DNA

<400>  2
tgcaagacta taagattc                                                  18

<210>  3
<211>  18
<212>  DNA
<213>  Artificial Sequence

<220>
<223>  PRNA-PNA-DNA chimera



<220>
<221>  misc_feature
<222>  (1)..(8)
<223>  peptide nucleic acid (PNA)

<220>
<221>  misc_feature
<222>  (9)..(9)
<223>  peptide ribonucleic acid (PRNA)

<220>
<221>  misc_feature
<222>  (11)..(11)
<223>  5'-amino-substituted DNA

<400>  3
tgcaagacua taagattc                                                    18


<210>  4
<211>  16
<212>  DNA
<213>  Artificial Sequence

<220>
<223>  PRNA-PNA-DNA chimera



<220>
<221>  misc_feature
<222>  (1)..(3)
<223>  peptide nucleic acid (PNA)

<220>
<221>  misc_feature
<222>  (4)..(4)
<223>  peptide ribonucleic acid (PRNA)

<220>
<221>  misc_feature
<222>  (5)..(10)
<223>  peptide nucleic acid (PNA)

<220>
<221>  misc_feature
<222>  (11)..(11)
<223>  5'-amino-substituted DNA

<400>  4
gtauatctcc ttctta                                                      16


<210>  5
<211>  16

<212>  DNA
<213>  Artificial Sequence

<220>
<223>  PRNA-PNA-DNA chimera



<220>
<221>  misc_feature
<222>  (1)..(3)
<223>  peptide nucleic acid (PNA)

<220>
<221>  misc_feature
<222>  (4)..(4)
<223>  peptide ribonucleic acid (PRNA)

<220>
<221>  misc_feature
<222>  (5)..(7)
<223>  peptide nucleic acid (PNA)

<220>
<221>  misc_feature
<222>  (8)..(8)
<223>  peptide ribonucleic acid (PRNA)

<220>
<221>  misc_feature
<222>  (9)..(10)
<223>  peptide nucleic acid (PNA)

<220>
<221>  misc_feature
<222>  (11)..(11)
<223>  5'-amino-substituted DNA

<400>  5
aaguactuag cgtaag                                                        16


<210>  6
<211>  18
<212>  DNA
<213>  Artificial Sequence

<220>
<223>  PRNA-PNA-DNA chimera



<220>
<221>  misc_feature
<222>  (1)..(2)
<223>  peptide nucleic acid (PNA)

<220>
<221>  misc_feature
<222>  (3)..(3)
<223>  peptide ribonucleic acid (PRNA)

<220>
<221>  misc_feature
<222>  (4)..(10)

<223>  peptide nucleic acid (PNA)

<220>
<221>  misc_feature
<222>  (11)..(11)
<223>  5′-amino-substituted DNA

<400>  6
tgcaagacta taagattc                                                    18


<210>  7
<211>  18
<212>  DNA
<213>  Artificial Sequence

<220>
<223>  PRNA-PNA-DNA chimera


<220>
<221>  misc_feature
<222>  (1)..(7)
<223>  peptide nucleic acid (PNA)

<220>
<221>  misc_feature
<222>  (8)..(8)
<223>  peptide ribonucleic acid (PRNA)

<220>
<221>  misc_feature
<222>  (9)..(10)
<223>  peptide nucleic acid (PNA)

<220>
<221>  misc_feature
<222>  (11)..(11)
<223>  5′-amino-substituted DNA

<400>  7
tgcaagacta taagattc                                                    18


<210>  8
<211>  21
<212>  DNA
<213>  Artificial Sequence

<220>
<223>  PRNA-PNA-DNA chimera


<220>
<221>  misc_feature
<222>  (1)..(7)
<223>  peptide nucleic acid (PNA)

<220>
<221>  misc_feature
<222>  (8)..(8)
<223>  peptide ribonucleic acid (PRNA)

<220>
<221>  misc_feature
<222>  (9)..(10)
<223>  peptide nucleic acid (PNA)

<220>
<221>  misc_feature
<222>  (11)..(11)
<223>  5'-amino-substituted DNA

<400>  8
tgcaagacta taagattcaa t                                                21


<210>  9
<211>  16
<212>  DNA
<213>  Artificial Sequence

<220>
<223>  PRNA-PNA-DNA chimera


<220>
<221>  misc_feature
<222>  (1)..(3)
<223>  peptide nucleic acid (PNA)

<220>
<221>  misc_feature
<222>  (4)..(4)
<223>  peptide ribonucleic acid (PRNA)

<220>
<221>  misc_feature
<222>  (5)..(8)
<223>  peptide nucleic acid (PNA)

<220>
<221>  misc_feature
<222>  (9)..(9)
<223>  5'-amino-substituted DNA

<400>  9
gtauatctcc ttctta                                                      16


<210>  10
<211>  18
<212>  DNA
<213>  Artificial Sequence

<220>
<223>  DNA-PRNA-PNA chimera


<220>
<221>  misc_feature
<222>  (9)..(10)
<223>  peptide nucleic acid (PNA)

<220>
<221>  misc_feature

<222>  (11)..(11)
<223>  peptide ribonucleic acid (PRNA)

<220>
<221>  misc_feature
<222>  (12)..(18)
<223>  peptide nucleic acid (PNA)

<400>  10
tgcaagacta uaagattc                                                    18


<210>  11
<211>  16
<212>  DNA
<213>  Artificial Sequence

<220>
<223>  DNA-PRNA-PNA chimera


<220>
<221>  misc_feature
<222>  (9)..(13)
<223>  peptide nucleic acid (PNA)

<220>
<221>  misc_feature
<222>  (14)..(14)
<223>  peptide ribonucleic acid (PRNA)

<220>
<221>  misc_feature
<222>  (15)..(16)
<223>  peptide nucleic acid (PNA)

<400>  11
gtatatctcc ttcuta                                                      16


<210>  12
<211>  18
<212>  RNA
<213>  Artificial Sequence

<220>
<223>  5′-FAM-RNA

<400>  12
gaaucuuaua gucuugca                                                    18


<210>  13
<211>  18
<212>  DNA
<213>  Artificial Sequence

<220>
<223>  anti-sense DNA

<400>  13
tgcaagacta taagattc                                                    18

<210>  14
<211>  16
<212>  RNA
<213>  Artificial Sequence

<220>
<223>  5′-FAM-RNA

<400>  14
uaagaaggag auauac                                                        16


<210>  15
<211>  16
<212>  DNA
<213>  Artificial Sequence

<220>
<223>  anti-sense DNA

<400>  15
gtatatctcc ttctta                                                        16